# EUROPÄISCHE PATENTSCHRIFT

(11) **EP 0 393 350 B1**
(45) Veröffentlichungstag und Bekanntmachung des Hinweises auf die Patenterteilung: **26.05.1993**
(21) Anmeldenummer: 90104779.5
(22) Anmeldetag: 14.03.1990
(51) Int. Cl.: C07C 209/50, C07C 211/35, C07C 229/56, C07C 209/58

(54) **Verfahren zur Herstellung von Aminen**
Process for the preparation of amines
Procédé pour la préparation d'amines

(30) Priorität: 21.03.1989 DE 3909142
(43) Veröffentlichungstag der Anmeldung: 24.10.1990
(73) Patentinhaber: BASF Aktiengesellschaft, 67063 Ludwigshafen (DE)
(72) Erfinder: Jones, Simon A., Dr., D-6906 St. Ilgen (DE); Jersak, Ulrich, Dr., D-6708 Neuhofen (DE)

(56) Entgegenhaltungen:
- EP-A- 0 205 403
- DE-A- 1 950 281
- FR-A- 2 093 472
- US-A- 4 082 749
- ADAMS: "ORGANIC REACTIONS" no. 3, 1946, JOHN WILEY, LONDON
- PATENT ABSTRACTS OF JAPAN vol. 1, no. 149 () 30 November 1977; & JP-A-52 093 723
- CHEMICAL ABSTRACTS, vol. 106, no. 25, 22 Juni 1987, Columbus, Ohio, USA; M. TAKAYAMA et al: "PROCESS FOR THE PREPARATION OF ANILINE DERIVATIVES BY HOFMANN REARRANGEMENT", & JP-A-61271255

## Beschreibung

Die vorliegende Erfindung betrifft ein verfahren zur Herstellung von Aminen aus Carbonsäureamiden oder deren Salzen mit Hypohalogenit in alkalischem Medium durch Vereinigung der Komponenten bei 60 bis 200°C.

Aus Houben-Weyl, Methoden der Organischen Chemie, Bd. 11/1, Seite 854 bis 862 (1957) sind allgemeine Methoden zur diskontinuierlichen Herstellung von Aminen aus Carbonsäureamiden (Hofmann-Abbau) mit Hypochlorit bekannt, wobei die Säureamide bei Temperaturen unterhalb Raumtemperatur - sehr häufig sogar unterhalb 0°C - mit alkalischer, wäßriger Hypochloritlösung zusammengebracht werden. Danach läßt man einige Zeit bei der gewählten tiefen Temperatur nachreagieren. Entweder durch Anwärmen oder durch die freiwerdende Reaktionswärme werden solche Reaktionsmischungen auf 50 bis 80°C gebracht und reagieren innerhalb von 30 bis 60 Minuten aus. Die alkalische Hypochloritlösung wird durch Einleiten von Chlor in Alkalilauge unter Kühlung in einem vorgelagerten Reaktionsschritt hergestellt. Der Hofmann-Abbau mit Hypobromitlösungen verläuft analog.

Aus der DE-A-19 50 281 ist ein kontinuierliches Verfahren zur Herstellung von Anthranilsäure bzw. Isatosäureanhydrid durch Vermischung von wäßrigen Phthalamidsäure-Alkalisalz-Lösungen mit Hypohalogeniten bei Raumtemperatur und anschließende adiabatische Reaktionsführung in einem Reaktionsrohr bekannt, wobei bestimmte Temperaturgrenzen für eine optimale Reaktionsführung wesentlich sind.

Diese Methoden lassen in ihren Aminausbeuten zu wünschen übrig und/oder sind in ihren Raum/Zeit-Ausbeuten noch verbesserungsfähig.

Der Erfindung lag daher die Aufgabe zugrunde, den zuvor genannten Nachteilen abzuhelfen.

Demgemäß wurde ein neues und verbessertes Verfahren zur Herstellung von Aminen aus Carbonsäureamiden oder deren Salzen mit Hypohalogenit in alkalischem Medium gefunden, welches dadurch gekennzeichnet ist, daß man die Komponenten bei 45 bis 260°C vereinigt.

Das erfindungsgemäße Verfahren läßt sich wie folgt durchführen.

Das alkalische Medium, das Hypohalogenit und das Carbonsäureamid können einzeln zusammengeführt werden oder das alkalische Medium mit dem Hypohalogenit zuvor vereinigt und dann mit dem Carbonsäureamid zur Reaktion gebracht werden, wobei in allen Fällen die Ausgangsmaterialien entweder durch Erhitzen oder vorzugsweise aus der Reaktionswärme bei der Herstellung des Hypohalogenits oder der Zusammenführung des Hypohalogenits mit dem alkalischen Medium vor der Reaktion mit dem Carbonsäureamid auf Temperaturen von 45 bis 260°C, vorzugsweise 60 bis 180°C gebracht werden. Man kann bei 0,1 bis 50 bar, vorzugsweise 0,2 bis 10 bar, besonders bevorzugt bei Atmosphärendruck arbeiten. Das Carbonsäureamid wird vorzugsweise in einem inerten Lösungsmittel vorgelegt.

Die Reaktion läßt sich diskontinuierlich oder vorzugsweise kontinuierlich durchführen.

Das Molverhältnis der Base im alkalischem Medium, bezogen auf das Carbonsäureamid, beträgt 1,7:1 bis 50:1, bevorzugt von 2:1 bis 20:1, besonders bevorzugt 2,1:1 bis 10:1.

Das Molverhältnis von Hypohalogenit zum Carbonsäureamid beträgt von 0,7:1 bis 30:1, bevorzugt von 1:1 bis 10:1, besonders bevorzugt 1:1 bis 3:1.

Als inerte Lösungsmittel für die Carbonsäureamide eignen sich Benzol, Toluol, Xylol, Chlorbenzol, Dichlorbenzol, Dichlormethan, Dichlorethan, Trichlorethan, Phenoxyethanol, bevorzugt aliphatische und cycloaliphatische Alkohole sowie Glykole und deren Mono- oder Diether, z.B. Methanol, Ethanol, n- und i-Propanol, n-, i- und s-Butanol, Cyclohexanol, Glykol, Methyl-, Ethyl-, Propyl- und Butylglykol, Methyldi- und Methyltriglykol, Ethyldi- und Ethyltriglykol, Butyldi- und Butyltriglykol, Propylglykol-1,2 und Propylglykol-1,3 oder Tetraglykol, besonders bevorzugt Wasser.

Die Menge an Lösungsmittel und/oder Wasser soll möglichst niedrig gehalten werden.

Als alkalische Medien eignen sich Alkalihydroxyde wie LiOH, NaOH und KOH in Form ihrer alkoholischen Lösung, vorzugsweise aber in Form ihrer 10 bis 90 Gew.% wäßrigen Lösungen, besonders bevorzugt als 30 bis 60 Gew.% wäßrige Lösungen, wie Natriumhydroxidlösung, Kaliumhydroxidlösung oder Lithiumhydroxidlösung.

Als Hypohalogenite eignen sich vor allem Hypochlorite und Hypobromite, z.B. Alkalihypochlorite und Alkalihypobromite wie Natriumhypochlorit, Kaliumhypochlorit, Natriumhypobromit und Kaliumhypobromit, bevorzugt in Form ihrer wäßrigen Lösungen.

Vorteile des erfindungsgemäßen Verfahrens sind:
- Verkürzung der Reaktionszeit,
- bessere Nutzung der freiwerdenden Reaktionswärme auch bei der Herstellung der Produkte,
- höhere Ausbeute und Qualität.

Als Carbonsäureamide eignen sich Alkyl-, Alkenyl-, Alkinyl-, Cycloalkyl-, Aryl-, durch Alkyl, Aralkyl, Aryl, Alkoxy, Chlor, Brom, Fluor, Hydroxycarboxy, Aryl substituierte Aryl- oder Arylalkyl-, durch Alkyl, Alkoxy, Fluor, Chlor, Brom, Hydroxy substituierte Arylalkyl-Carbonsäureamide wie Acetamid, Methoypropionsäureamid, Propionamid, Phenylessigsäureamid, Benzoesäureamid, o-, m-, p-Chlorbenzoesäureamid, o-, m-, p-Toluolcarbonsäurecyclohexancarbonsäureamid, bevorzugt aber Cyclopropancarbonsäureamid und Phthalamidsäure.

Aus den Carbonsäureamiden sind folgende Amine erhältlich:
Alkyl-, Alkenyl-, Alkinyl-, Cycloalkyl-, Aryl-, durch Alkyl, Aralkyl, Aryl, Alkoxy substituierte Aryl- oder Arylalkyl-, durch Alkyl, Alkoxy, Fluor, Chlor, Brom, Hydroxy substituierte Arylalkylamine wie Methylamin, β-Methoxyethylamin, Ethylamin, Benzylamin, Anilin, o-, m-, p-Chloranilin, o-, m-, p-Tolylamin, Vinylamin, Cyclohexylamin, bevorzugt Cyclopropanamin, Isatosäureanhydrid und Anthranilsäure.

Die nach dem erfindungsgemäßen Verfahren erhältlichen Amine sind wertvolle Zwischenprodukte für Farbstoffe (Fierz-David, Grundlagen der Farbenchemie), Kunststoffe oder für Wirkstoffe aus dem Pflanzenschutz oder für Pharmazeutika.

### Beispiele

### Beispiel 1

In eine Mischdüse mit nachgeschaltetem Rohrreaktor wurden vermischt:
- 13,4 kg/h: 8 %ige wäßrige Lösung von Cyclopropancarbonsäureamid, vorgewärmt auf 90°C 10,3 kg/h alkalische Bleichlaugelösung
- (T = 18°C),: die in einer Vorlage aus 12 kg 50 %iger wäßriger Natronlauge und 39 kg Bleichlauge (13 % aktives Chlor) hergestellt und bei 18°C gelagert wurde.

- Mischtemperatur nach der Düse:: 70°C
- Temperatur am Reaktorausgang:: 98°C
- Verweilzeit im Reaktor:: 30 sec.

Nach destillativer Aufarbeitung des Reaktoraustrages erhielt man 0,66 kg/h Cyclopropanamin 99,5 %ig. Das entspricht einer Ausbeute von 92 % der Theorie.

### Beispiel 2

Analog Beispiel 1 - nur wurden an Stelle der alkalischen Bleichlauge 50 %ige Natronlauge und Bleichlauge (13 % akt. Chlor) vor Eintritt in die Mischdüse vorvermischt
13,4 kg/h 8 %ige Cyclopropancarbonsäureamidlösung T = 80°C 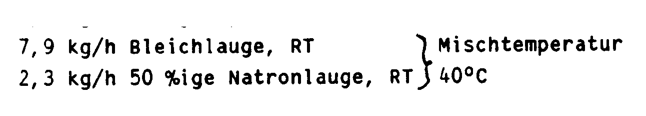
- Mischtemperatur nach Düse:: 75°C
- Temperatur am Reaktorausgang:: 95°C
- Verweilzeit im Reaktor:: 30 sec.

Ausbeute wie in Beispiel 1.

### Beispiel 3

In einer Mischdüse mit nachgeschaltetem Rohrreaktor wurden vermischt: 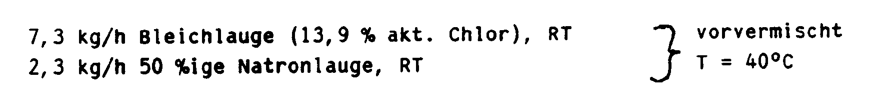
13,4 kg/h Phthalamidsäure-Na-Salz, 20 %ig in Wasser, T = 75°C
- Mischtemperatur nach Düse:: 107°C
- Reaktorausgang:: 100°C
- Verweilzeit:: 15 sec.

Der Reaktoraustrag wurde gesammelt und diskontinuierlich mit Salzsäure versetzt zum Zwecke der Ausfällung der Anthranilsäure.

3 kg Reaktoraustrag wurden bei 20°C mit 0,34 kg 33 %iger Salzsäure versetzt. pH = 4,2. Der Niederschlag wurde abfiltriert, ausgewaschen und getrocknet. Man erhielt 0,223 kg Anthranilsäure 99 %ig. Ausbeute 87 % der Theorie.

### Beispiel 4

In einer Mischdüse mit nachgeschaltetem Rohrreaktor wurden vermischt:
5,8 kg/h Bleichlauge (13,0 % akt. Chlor), RT
22,4 kg/h Phthalamidsäure-Na-Salz, 8,8 %ig in Wasser,
pH 18°C = 12,0, T = 40°C mit 4 Gew.-% Amidosulfonsäure-Na-Salz als Katalysator
- Mischtemperatur nach Düse:: 45°C
- Reaktorausgang:: ca. 50°C
- Verweilzeit:: 2 sec.

Der Reaktoraustrag wurde kontinuierlich mit Salzsäure bei pH = 6,1, T = 50°C versetzt zur Ausfällung des Isatosäureanhydrids. Der Niederschlag wird abfiltriert, ausgewaschen und getrocknet. Man erhielt Isatosäureanhydrid 99,8 %ig mit einer Ausbeute >90 % der Theorie.

## Patentansprüche

1. Verfahren zur Herstellung von Aminen aus Carbonsäureamiden oder deren Salzen mit Hypohalogenit in alkalischem Medium, dadurch gekennzeichnet, daß man die Komponenten bei 45 bis 260°C vereinigt.

2. Verfahren nach Anspruch 1, dadurch gekennzeichnet, daß man die Komponenten bei 60 bis 180°C vereinigt.

3. Verfahren nach Anspruch 1, dadurch gekennzeichnet, daß man in wäßrigem alkalischem Medium arbeitet.

4. Verfahren nach Anspruch 1, dadurch gekennzeichnet, daß man als Hypohalogenit Hypochlorit oder Hypobromit verwendet.

5. Verfahren nach Anspruch 1, dadurch gekennzeichnet, daß man als alkalisches Medium wäßriges Alkalihydroxid verwendet.

6. Verfahren nach Anspruch 1, dadurch gekennzeichnet, daß man als Hypohalogenit ein Alkalihypochlorit oder ein Alkalihypobromit verwendet.

7. Verfahren nach Anspruch 1 bis 6, dadurch gekennzeichnet, daß man die Umsetzung kontinuierlich durchführt.

## Claims

1. A process for the preparation of amines from carboxamides or salts thereof with hypohalite in alkaline medium, which comprises combining the components at 45 to 260°C.

2. A process as claimed in claim 1, wherein the components are combined at 60 to 180°C.

3. A process as claimed in claim 1, which is carried out in aqueous alkaline medium.

4. A process as claimed in claim 1, wherein hypochlorite or hypobromite is used as hypohalite.

5. A process as claimed in claim 1, wherein aqueous alkali metal hydroxide is used as alkaline medium.

6. A process as claimed in claim 1, wherein hypochlorite or an alkali metal hypobromite is used as hypohalite.

7. A process as claimed in claim 1 to 6, wherein the reaction is carried out continuously.

## Revendications

1. Procédé de préparation d'amines à partir d'acides carboxyliques ou de leurs sels avec un hypohalogénite en milieu alcalin, caractérisé en ce que l'on réunit les composants à 45-260°C.

2. Procédé selon la revendication 1, caractérisé en ce que l'on réunit les composants à 60-180°C.

3. Procédé selon la revendication 1, caractérisé en ce que l'on travaille en milieu aqueux alcalin.

4. Procédé selon la revendication 1, caractérisé en ce que l'on utilise un hypochlorite ou un hypobromite à titre d'hypohalogénite.

5. Procédé selon la revendication 1, caractérisé en ce que l'on utilise un hydroxyde de métal alcalin aqueux à titre de milieu aqueux alcalin.

6. Procédé selon la revendication 1, caractérisé en ce que l'on utilise un hypochlorite de métal alcalin ou un hypobromite de métal alcalin à titre d'hypohalogénite.

7. Procédé selon l'une quelconque des revendications 1 à 6, caractérisé en ce que l'on entreprend la réaction en continu.
